# EUROPEAN PATENT APPLICATION

(11) **EP 3 100 744 A1**
(43) Date of publication of application: **07.12.2016**
(21) Application number: 15744038.9
(22) Date of filing: 28.01.2015
(51) Int. Cl.: A61K 47/22, A61K 9/70, A61K 31/192

(54) **COMPOSITION FOR ACCELERATING PENETRATION THROUGH SKIN, PREPARATION FOR TRANSDERMAL ADMINISTRATION, AND SKIN PATCH PREPARATION**

(30) Priority: 29.01.2014 JP 2014014539
(71) Applicant: Nitto Denko Corporation, Ibaraki-shi, Osaka 567-8680 (JP)
(72) Inventor: MAEDA, Yoshiki, Ibaraki-shi Osaka 567-8680 (JP); MATSUSHITA, Kyohei, Ibaraki-shi Osaka 567-8680 (JP); LI, Wenjing, Ibaraki-shi Osaka 567-8680 (JP); OKUBO, Katsuyuki, Ibaraki-shi Osaka 567-8680 (JP); HORI, Mitsuhiko, Ibaraki-shi Osaka 567-8680 (JP)
(74) Representative: Müller-Boré & Partner Patentanwälte PartG mbB
(86) International application number: PCT/JP2015/052388
(87) International publication number: WO 2015/115495

(57) **Abstract**

The present invention provides a skin penetration-accelerating composition that eliminates the need for forming a drug into a particle structure and dramatically improves the skin penetration properties of a drug without breaking the skin tissue. The present invention also provides a preparation for transdermal administration containing the skin penetration-accelerating composition and a patch preparation containing the skin penetration-accelerating composition. The present invention provides a skin penetration-accelerating composition containing a flavonoid compound and used for accelerating skin penetration of a drug.

## Description

### TECHNICAL FIELD

The present invention relates to a skin penetration-accelerating composition used for accelerating skin penetration of a drug. In particular, the present invention relates to a skin penetration-accelerating composition that eliminates the need for forming a drug into a particle structure and dramatically improves skin penetration properties of a drug without breaking the skin tissue. The present invention also relates to a preparation for transdermal administration containing the skin penetration-accelerating composition and a patch preparation containing the skin penetration-accelerating composition.

### BACKGROUND ART

A preparation for transdermal administration is a dosage form receiving attention in efforts to achieve noninvasive administration, avoid the hepatic first-pass effect, and reduce side effects on the alimentary canal.

In transdermal administration, the principal drug needs to penetrate the skin tissue, the strongest biological barrier, to exhibit its efficacy. Various drug penetration accelerators have been used. Known preparations used in transdermal administration include: a transdermal absorption-type patch (Patent Literature 1) containing, as a penetration accelerator, an alkanolamine, a surfactant, an alcohol or an ester or ether thereof, a fatty acid or an ester thereof, or a terpene; and a transdermal absorption-type patch (Patent Literature 2) containing crotamiton as a penetration accelerator.

However, even the use of these penetration accelerators is insufficient for many bioactive drugs to exhibit accelerated skin penetration. If these penetration accelerators are used in amounts sufficient for enough accelerating effects, the skin tissue breaks. It is thus very difficult to improve skin penetration properties of a drug.

Several other techniques have been proposed, such as breaking a part of the skin structure with a microneedle or the like (Patent Literature 3) or forming a drug into fine particles with an average particle size below a predetermined value to make it easy for the drug to pass through gaps in the corneum (Patent Literature 4).

Use of a conventional strong penetration accelerator or breaking of a part of the skin structure with a microneedle or the like cause significant change in the skin structure if higher accelerating effects are desired. These techniques are thus undesirable in systems where the normal functions of the skin are required for therapeutic effects. These methods in addition may cause side reactions such as skin irritation. As for forming a drug into particles, only limited drugs can be formed into particles. Additionally, the structure or the activity of the drug may be broken during the particle formation process.

### CITATION LIST

### - Patent Literature

Patent Literature 1: JP 2013-79220 A
Patent Literature 2: JP H10-279474 A
Patent Literature 3: JP 5032121 B
Patent Literature 4: JP 2012-206979 A

### SUMMARY OF INVENTION

### - Technical Problem

In view of the situation in the art, the present invention aims to provide a skin penetration-accelerating composition that eliminates the need for forming a drug into a particle structure and dramatically improves skin penetration properties of a drug without breaking the skin tissue. The present invention also aims to provide a preparation for transdermal administration containing the skin penetration-accelerating composition and a patch preparation containing the skin penetration-accelerating composition.

### - Solution to Problem

To solve the above problems, the present inventors focused on the use of a flavonoid compound as a drug penetration accelerator. Some effects of flavonoid compounds on the skin have been reported. For example, JP 2008-260747 A discloses that flavonoid compounds exert a protective effect on the skin by accelerating collagen production. However, their effect on the skin penetration properties of bioactive drugs has not been reported.

After intensive studies, the present inventors found that adding a flavonoid compound along with a drug to a preparation for transdermal administration remarkably accelerates the skin penetration properties of the drug, causing almost no irritation or damage to the skin. The inventors thus completed the present invention.

The present invention is directed to a skin penetration-accelerating composition containing a flavonoid compound, the skin penetration-accelerating composition being used for accelerating skin penetration of a drug.

The flavonoid compound is preferably a compound having a flavone skeleton represented by Formula (1).

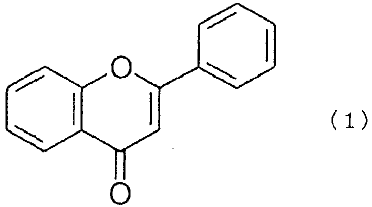

The compound having a flavone skeleton represented by Formula (1) is preferably at least one compound selected from the group consisting of quercetin, apigenin, and myricetin.

The present invention is also directed to a preparation for transdermal administration containing a drug and the skin penetration-accelerating composition of the present invention.

The present invention is also directed to a patch preparation including a drug-containing layer that contains a drug and the skin penetration-accelerating composition of the patch preparation.

The present invention will be described in detail below.

The present invention is directed to a skin penetration-accelerating composition containing a flavonoid compound. The composition is used for accelerating skin penetration of a drug.

The skin penetration-accelerating composition of the present invention contains a flavonoid compound and thus can remarkably improve the skin penetration properties of a drug without breaking the skin tissue.

The flavonoid compound may be, for example, a compound having a flavone skeleton, a compound having an isoflavone skeleton, or a catechin compound. In particular, the flavonoid compound is preferably a compound having a flavone skeleton represented by Formula (1).

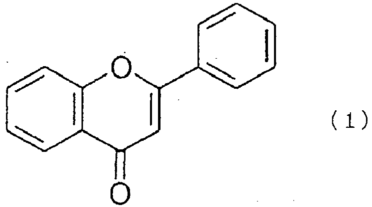

Any compound having a flavone skeleton represented by Formula (1) may be used. Suitable examples include flavone, flavonol, flavanone, hydroxyflavone, methoxyflavone, dihydroxyflavone, baicalein, trihydroxyflavone, hydroxyflavonol, galangin, fisetin, morin, isorhamnetin, robinetin, quercetagetin, naringenin, naringin, eriodictyol, hesperetin, formononetin, biochanin A, apigenin, chrysin, cyanidin, hesperidin, kaempferol, myricetin, nobiletin, quercetin, rutin, sulfuretin, tangeretin, luteolin, and pharmaceutically acceptable salts thereof. These compounds having a flavone skeleton may be used alone or in combination of two or more. The compound having a flavone skeleton represented by Formula (1) is preferably at least one compound selected from the group consisting of quercetin, apigenin, and myricetin.

Any compound having an isoflavone skeleton may be used. Examples thereof include isoflavone, genistein, daidzin, genistin, glycitin, daidzein, glycitein, 6"-O-acetyldaidzin, 6"-O-acetylgenistin, 6"-O-acetylglycitin, 6"-O-malonyldaidzin, 6"-O-malonylgenistin, and 6"-O-malonylglycitin. These compounds having an isoflavone skeleton may be used alone or in combination of two or more.

Any catechin compound may be used. Examples thereof include catechin, epicatechin, gallocatechin, epigallocatechin, catechin gallate, epicatechin gallate, gallocatechin gallate, and epigallocatechin gallate. These catechin compounds may be used alone or in combination of two or more.

The skin penetration-accelerating composition of the present invention may further contain a surfactant. Use of the flavonoid compound in combination with a surfactant further improves the skin penetration properties of a drug.

Any surfactant may be used. Examples thereof include ionic surfactants and nonionic surfactant.

The use of the skin penetration-accelerating composition of the present invention is not limited as long as it is the use of accelerating skin penetration of a drug. The skin penetration-accelerating composition of the present invention is preferably blended with a drug and used for transdermal administration.

A preparation for transdermal administration containing a drug and the skin penetration-accelerating composition of the present invention is encompassed by the present invention.

The amount of the flavonoid compound in the preparation for transdermal administration of the present invention is preferably 0.1 to 10,000 parts by weight based on 100 parts by weight of the drug from the viewpoint of skin penetration-accelerating effect of the drug. The lower limit of the amount of the flavonoid compound is more preferably 1 part by weight, and the upper limit thereof is more preferably 1000 parts by weight. The lower limit is still more preferably 10 parts by weight.

Any drug may be used. Examples thereof include sedatives, expectorants, laxatives, anti-cancer drugs, anti-diabetic drugs, anti-parkinsonian drugs, antidepressants, tranquilizers, dementia drugs, hypotensive drugs, hyperlipemia drugs, migraine drugs, drugs for treatment of osteoporosis, drugs for treatment of hypotension, antitussives, peptic ulcer drugs, pollakisuria/dysuria drugs, urinary incontinence drugs, anti-ulcer drugs, antiallergic drugs, and 5-HT3 receptor antagonists (antiemetic drugs), antigenic peptides, antigenic protein, and nucleic acid. The drug is preferably a drug causing no skin irritation. However, even a drug causing skin irritation can be suitably used by combining it with an anti-inflammatory agent. Among these drugs, acidic drugs and drugs forming a water-soluble salt are more suitable, because such drugs are less likely to interact with phenolic hydroxy groups abundantly present in the flavonoid compounds and thus are less likely to inhibit the effects of the flavonoid compounds.

The preparation for transdermal administration of the present invention may be in any dosage form. Examples thereof include ointment, cream, liquid, lotion, liniment, cataplasm, plaster, and patch (e.g., film, tape). Among these dosage forms, patch is preferred.

A patch preparation including a drug-containing layer that contains a drug and the skin penetration-accelerating composition of the present invention is also encompassed by the present invention.

The amount of the flavonoid compound in the drug-containing layer is not limited. The amount is preferably 0.01 to 80% by weight in the total weight of the solids contained in the drug-containing layer. If the amount is less than 0.01% by weight, the flavonoid compound may not reach the skin, failing to sufficiently accelerate the skin penetration of a drug. If the amount is more than 80% by weight, the drug-containing layer may not retain the flavonoid compound, making it difficult to apply the patch preparation to the surface of the skin. The lower limit of the amount is more preferably 0.05% by weight, the upper limit thereof is more preferably 30% by weight.

The drug may be dissolved or dispersed into the drug-containing layer.

The amount of the drug in the drug-containing layer is not limited, and may vary depending on factors such as the characteristics of the drug used. The amount of the drug is preferably 0.01 to 60% by weight in the total weight of the solids contained in the drug-containing layer. If the amount is less than 0.01% by weight, the drug may not reach the skin, failing to exhibit sufficient efficacy. If the amount is more than 60% by weight, the drug-containing layer may not retain the drug, making it difficult to apply the patch preparation to the surface of the skin.

The thickness of the drug-containing layer is not limited. The thickness is preferably 10 to 1000 µm, more preferably 20 to 500 µm, still more preferably 30 to 200 µm. If the thickness of the drug-containing layer is less than 10 µm, it may be difficult for the drug-containing layer to contain the drug and the flavonoid compound in effective amounts, or it may be difficult for the drug-containing layer to have sufficient adhesion when the layer requires adhesiveness. If the thickness of the drug-containing layer is more than 1000 µm, the drug-containing layer may be difficult to form (coating difficulty).

The patch preparation of the present invention is preferably a patch preparation (so-called matrix-type patch preparation) in which the drug-containing layer is a drug-containing adhesive layer and the drug-containing adhesive layer is on one surface of a support. The patch preparation of the present invention may be a patch preparation (so-called reservoir-type patch preparation) in which the drug-containing layer is a drug storage layer and the drug storage layer and an adhesive layer are on one surface of a support. For a reservoir-type patch preparation, a drug penetration-controlling membrane is preferably further provided between the drug storage layer and the adhesive layer.

A release liner may be disposed on the drug-containing adhesive layer or on the adhesive layer for purposes such as protection and storage stability.

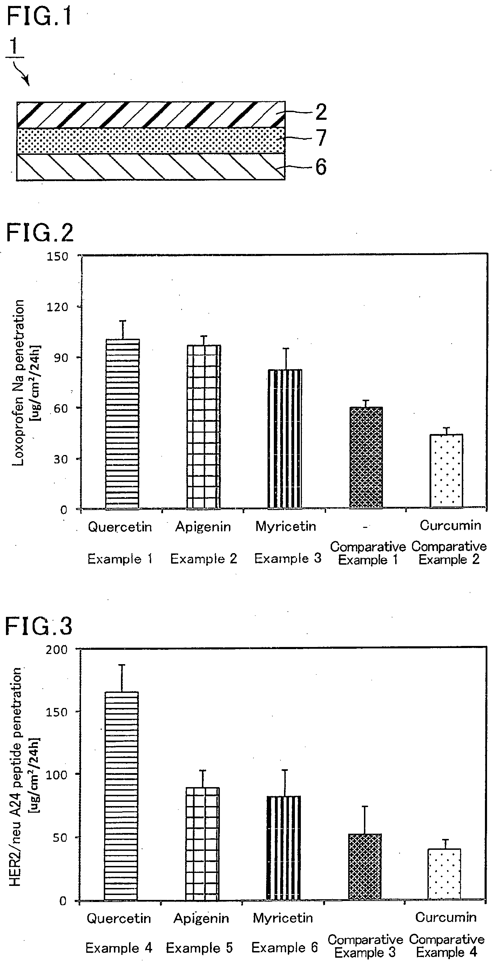

Fig. 1 is a schematic cross-sectional view of an example of the patch preparation (matrix-type patch preparation) of the present invention. As shown in Fig. 1, in a patch preparation (matrix-type patch preparation) 1 according to the present invention, a drug-containing adhesive layer 7 containing a drug and the skin penetration-accelerating composition of the present invention and a release liner 2 are layered on one surface of a support 6.

The resin constituting the drug-containing adhesive layer is preferably an adhesive polymer.

Any adhesive polymer may be used. Examples thereof include acrylic polymers including (meth)acrylate polymers; rubber polymers such as styrene-isoprene-styrene block copolymers, styrene-butadiene-styrene block copolymers, polyisoprene, polyisobutylene, and polybutadiene; silicone polymers such as silicone rubber, dimethylsiloxane-based polymers, and diphenylsiloxane-based polymers; vinyl ether polymers such as polyvinyl methyl ether, polyvinyl ethyl ether, and polyvinyl isobutyl ether; vinyl ester polymers such as vinyl acetate-ethylene copolymers; and ester polymers composed of a carboxylic acid component such as dimethyl terephthalate, dimethyl isophthalate, or dimethyl phthalate and a polyalcohol component (e.g., ethylene glycol). Among these polymers, rubber polymers are preferred because they have excellent retentivity of the flavonoid compound.

The acrylic polymer is preferably an acrylic polymer obtained by copolymerizing alkyl (meth)acrylate, as a main component, with a functional monomer. Specifically, The acrylic polymer is preferably a copolymer in which a monomer component consisting of the alkyl (meth) acrylate is present in an amount of 50 to 99% by weight (more preferably 60 to 95% by weight) and the rest of the monomer components consist of a functional monomer. The main component herein means a monomer component present in an amount of 50% by weight or more of the total weight of the monomer components constituting the copolymer.

The alkyl (meth)acrylate preferably contains a C4-C13 straight-chain or branched alkyl group (e.g., butyl, pentyl, hexyl, heptyl, octyl, 2-ethylhexyl, nonyl, decyl, undecyl, dodecyl, tridecyl) as an alkyl group. These alkyl (meth)acrylates may be used alone or in combination of two or more.

The functional monomer has, in the molecule, at least one unsaturated double bond that is involved in copolymerization, and has a functional group in a side chain. Examples of the functional monomer include carboxy group-containing monomers such as (meth)acrylic acid, itaconic acid, maleic acid, and maleic anhydride, hydroxy group-containing monomers such as hydroxyethyl (meth)acrylate and hydroxypropyl (meth)acrylate; sulfoxy-group containing monomers such as styrenesulfonic acid, allylsulfonic acid, sulfopropyl (meth)acrylate, (meth)acryloyloxynaphthalenesulfonic acid, and acrylamidemethylpropanoic acid; amino group-containing monomers such as aminoethyl (meth)acrylate, dimethylaminoethyl (meth)acrylate, and tert-butylaminoethyl (meth)acrylate; amide group-containing monomers such as (meth)acrylamide, dimethyl(meth)acrylamide, N-methylol(meth)acrylamide, N-methylolpropane(meth)acrylamide, and N-vinylacetamide; and alkoxy group-containing monomers such as methoxyethyl (meth)acrylate, ethoxyethyl (meth)acrylate, methoxy ethylene glycol (meth)acrylate, methoxy diethylene glycol (meth)acrylate, methoxy polyethylene glycol (meth)acrylate, methoxy polyprene glycol (meth)acrylate, and tetrahydrofuryl (meth)acrylate. These functional monomers may be used alone or in combination of two or more. Among these monomers, carboxy group-containing monomers are preferred and (meth) acrylic acid is particularly preferred, from the viewpoint of the pressure-sensitive adhesion and aggregability of the drug-containing adhesive layer and the releasability of the drug contained in the drug-containing adhesive layer.

For the acrylic polymer, the alkyl (meth) acrylate and the functional monomer may be further copolymerized with other monomer(s).

Examples of other monomers include (meth) acrylonitrile, vinyl acetate, vinyl propionate, N-vinyl-2-pyrrolidone, methylvinylpyrrolidone, vinylpyridine, vinylpiperidone, vinylpyrimidine, vinylpiperazine, vinylpyrrole, vinylimidazole, vinylcaprolactam, and vinyloxazole.

Such other monomers may be used alone or in combination of two or more.

The amount of such other monomers preferably usually around 0 to 40% by weight, more preferably around 10 to 30% by weight based on the total weight of the alkyl (meth)acrylate and the functional monomer.

Specifically, from the viewpoint of good adhesiveness to human skin and ease of repeating adhesion and release, the acrylic polymer is preferably a terpolymer of 2-ethylhexyl acrylate as alkyl (meth) acrylate, acrylic acid, and N-vinyl-2-pyrrolidone, more preferably a copolymer of 2-ethylhexyl acrylate, acrylic acid, and N-vinyl-2- pyrrolidone at a weight ratio of 40 to 99.8:0.1 to 10:0.1 to 50, preferably 50 to 89:1 to 8:10 to 40.

The rubber polymers is preferably a rubber polymer containing, as a main component, at least one selected from the group consisting of polyisobutylene, polyisoprene, and a styrene-diene-styrene block copolymer (e.g., styrene-butadiene-styrene block copolymer (SBS), styrene-isoprene-styrene block copolymer (SIS)). From the viewpoint of high drug stability and achieving both desired adhesion and aggregability, particularly preferred is a mixture containing a high-molecular-weight polyisobutylene having a viscosity average molecular weight of preferably 1,800,000 to 5,500,000, more preferably 2,000,000 to 5,000,000 and a low-molecular-weight polyisobutylene having a viscosity average molecular weight of preferably 40,000 to 85,000, more preferably 45,000 to 65,000, at a weight ratio of 95: 5 to 5: 95.

If the drug-containing adhesive layer contains the rubber polymer, the drug-containing adhesive layer preferably further contains a tackifier. Addition of a tackifier can improve the adhesiveness of the drug-containing adhesive layer at room temperature.

Any tackifier may be used. A known tackifier in the art may be appropriately selected. Examples of the tackifier include petroleum resins (e.g., aromatic petroleum resins, aliphatic petroleum resins), terpene resins, rosin resins, coumarone-indene resins, styrene resins (e.g., styrene resin, poly(α-methylstyrene)), hydrogenated petroleum resins (e.g., alicyclic saturated hydrocarbon resins). These tackifiers may be used alone or in combination of two or more. Among these tackifiers, alicyclic saturated hydrocarbon resins are suitable because good drug stability can be achieved.

The amount of the tackifier is usually preferably 33 to 300% by weight, more preferably 50 to 200% by weight based on the total weight of the rubber polymers.

The drug-containing adhesive layer preferably further contains a plasticizer.

Any plasticizer may be used as long as it can impart soft feel to the drug-containing adhesive layer by plasticizing the adhesive polymer and can reduce pain or skin irritation due to the skin adhesion upon peeling the patch preparation from the skin.

Examples of the plasticizer include fats and oils such as olive oil, castor oil, squalene, lanolin, organic solvents such as decylmethylsulfoxide, methyloctylsulfoxide, dimethylsulfoxide, dimethylformamide, dimethylacetamide, methylpyrrolidone, and dodecylpyrrolidone, surfactants such as sorbitan fatty acid esters and polyoxyethylene fatty acid esters, phthalates such as dibutyl phthalate, diheptyl phthalate, and dioctyl phthalate, sebacates such as diethyl sebacate, dibutyl sebacate, and dioctyl sebacate, hydrocarbons such as liquid paraffin, fatty acid esters such as ethyl oleate, diisopropyl adipate, isopropyl palmitate, octyl palmitate, isopropyl myristate, isotridecyl myristate, and ethyl laurate, glycerol fatty acid esters, propylene glycol fatty acid esters, ethoxylated stearyl alcohols, and pyrrolidone carboxylic acid fatty acid esters. These plasticizers may be used alone or in combination of two or more.

The amount of the plasticizer is preferably 1 to 70% by weight, more preferably 20 to 60% by weight in 100% by weight of the drug-containing adhesive layer.

A cross-linked structure may be introduced to the drug-containing adhesive layer. To introduce the cross-linked structure, the drug-containing adhesive layer may be subjected to a physical cross-linking treatment by radiation such as ultraviolet ray irradiation or electron ray irradiation, or a chemical cross-linking treatment using a cross-linking agent. Examples of the cross-linking agent include isocyanate compounds (e.g., trifunctional isocyanates), organic peroxides, organic metal salts, metal alcoholates, metal chelate compounds, multifunctional compounds (e.g., multifunctional external cross-linking agents, multifunctional monomers for internal crosslinking such as di(meth)acrylates).

In the case of the chemical crosslinking treatment, the cross-linking agent is incorporated into a drug-containing adhesive solution or dispersion with a drug and the skin penetration-accelerating composition of the present invention. The drug-containing adhesive solution or dispersion is applied to one surface of a support and dried to form a drug-containing adhesive layer. Thereafter, a release liner is applied such that its release-treated surface contacts the drug-containing adhesive layer. The drug-containing adhesive layer is allowed to stand for 24 to 48 hours at preferably 60°C to 90°C, more preferably 60°C to 70°C to promote crosslinking, whereby a drug-containing adhesive layer with a crosslinking structure is formed. Alternatively, the drug-containing adhesive solution or dispersion may be applied to the release-treated surface of a release liner and the formed drug-containing adhesive layer may be transferred to a support.

Any support may be used. Specific examples thereof include single films of polyesters (e.g., polyethylene terephthalate (PET)), nylons, polyvinyl chloride, polyethylene, polypropylene, ethylene-vinyl acetate copolymers, polytetrafluoroethylene, and ionomer resins, metallic foils, and laminate films including one or two or more selected from these films. In order to improve the adhesiveness (anchoring properties) between the support and the drug-containing adhesive layer, it is preferred that the support is a laminate film including a non-porous film made of any of the above materials and a porous film (described later), and that the drug-containing adhesive layer is formed on the porous film side. The non-porous film preferably has a thickness of 1 to 100 µm, more preferably 2 to 50 µm.

Any porous film may be used as long as it improves the anchoring properties between the support and the drug-containing adhesive layer. Examples thereof include paper, woven fabrics, non-woven fabrics (e.g., polyester (e.g., polyethylene terephthalate (PET)) non-woven fabrics), and films obtained by mechanically perforating films made of any of the above materials (e.g., single films of polyesters, nylons, saran (trade name), polyethylene, polypropylene, ethylene-vinyl acetate copolymers, polyvinyl chloride, ethylene-ethyl acrylate copolymers, polytetrafluoroethylene, metallic foils, or polyethylene terephthalate, laminate films including one or two or more films selected from these films). From the viewpoint of flexibility, paper, woven fabrics, non-woven fabrics (e.g., polyester non-woven fabrics, polyethylene terephthalate non-woven fabrics) are preferred.

If the porous film is, for example, a woven fabric or a non-woven fabric, the weight per unit area thereof is preferably 5 to 30 g/m² from the viewpoint of the anchoring properties.

The laminate film in the support may be formed by a conventional method for producing a laminate film. Examples thereof include a dry lamination method, a wet lamination method, an extrusion lamination method, a hot melt lamination method, a co-extrusion lamination method.

The thickness of the support is not limited. The thickness is preferably 1 to 200 µm, more preferably 2 to 100 µm. If the thickness of the support is less than 1 µm, the handling properties such as self-supporting properties tends to be low. If the thickness of the support is more than 200 µm, the support causes uncomfortable feel (rough feel) and tends to have low conformability.

The release liner may be a release liner in which a release-treated layer made of a release treatment agent is formed on a surface of a substrate for a release liner, a plastic film having high releasability by itself, or a release liner in which a release layer made of such a plastic film having high releasability is formed on a surface of a substrate for a release liner. The release surface of the release liner may be only one surface or both surfaces of the substrate.

Any release treatment agent may be used. Examples thereof include long chain alkyl group-containing polymers, silicone polymers (silicone release agents), and fluoropolymers (fluorine release agents).

Examples of the substrate for a release liner include plastic films such as polyethylene terephthalate (PET) films, polyimide films, polypropylene films, polyethylene films, polycarbonate films, and polyester (excluding PET) films, and metallized plastic films obtained by evaporating a metal on any of these films; paper such as Japanese paper, western paper, kraft, glassine, and woodfree paper; substrates made of fibrous materials such as non-woven fabrics, cloth; and metallic foils.

Examples of the plastic film having high releasability by itself include polyolefin films made of polyethylenes (e.g. , low-density polyethylene, linear low-density polyethylene), polypropylene, ethylene-α-olefin copolymers (block copolymers or random copolymers) such as ethylene-propylene copolymers, and mixtures thereof; and Teflon (registered trademark) films.

The release layer on a surface of the substrate for a release liner may be formed by laminating or coating a material for the plastic film having high releasability on a surface of the substrate for a release liner.

The thickness of the release liner is not limited. The thickness is usually preferably 200 µm or less, more preferably 25 to 100 µm.

The patch preparation of the present invention may be prepared by the following method, for example. First, a predetermined amount of an adhesive polymer is dissolved in a solvent (e.g., ethyl acetate, toluene, hexane, dimethylsulfoxide, ethanol, propanol, acetone). Next, a drug and the skin penetration-accelerating composition of the present invention are dispersed or dissolved into the obtained solution to prepare a drug-containing adhesive solution or dispersion. Then, the drug-containing adhesive solution or dispersion is applied to one surface of a support and dried to form a drug-containing adhesive layer. Thereafter, a release liner is applied such that its release-treated surface contacts the drug-containing adhesive layer. Alternatively, the drug-containing adhesive solution or dispersion may be applied to the release-treated surface of a release liner and the formed drug-containing adhesive layer may be transferred to a support.

If bubbles are produced during preparing the drug-containing adhesive solution or dispersion, the solution or dispersion is preferably allowed to stand overnight or subj ected to vacuum defoamation. The drug-containing adhesive solution or dispersion may be applied to one surface of a support or a release liner by, for example, casting, printing, or other known technique in the art.

### - Advantageous Effects of Invention

The present invention provides a skin penetration-accelerating composition that eliminates the need for forming a drug into a particle structure and drastically improves skin penetration properties of a drug without breaking the skin tissue. The present invention also provides a preparation for transdermal administration containing the skin penetration-accelerating composition and a patch preparation containing the skin penetration-accelerating composition.

### BRIEF DESCRIPTION OF DRAWINGS

Fig. 1 is a schematic cross-sectional view of an example of the patch preparation (matrix-type patch preparation) of the present invention.
Fig. 2 shows the results of a skin penetration property test on patch preparations obtained in examples and comparative examples.
Fig. 3 shows the results of a skin penetration property test on patch preparations obtained in examples and comparative examples.
Fig. 4 shows the results of a skin penetration property test on patch preparations obtained in examples and comparative examples.
Fig. 5 shows the results of a skin penetration property test on patch preparations obtained in examples and comparative examples.

### DESCRIPTION OF EMBODIMENTS

The present invention will be specifically described with reference to examples below. The present invention is not limited to these examples.

### (Examples 1 to 3)

A solution in toluene of a mixture of polyisobutylene and a tackifier (a mixture of 24 parts by weight of polyisobutylene B200 (viscosity average molecular weight: 4,000,000), 36 parts by weight of polyisobutylene B12 (viscosity average molecular weight: 55,000), and 40 parts by weight of alicyclic saturated hydrocarbon resin ARKON P-100, each in terms of the solid content; hereinafter referred to as PIB blend) was prepared. An amount of 63.0 parts by weight of the solution, calculated based on the solid content in the composition, was weighed. Subsequently, 27.0 parts by weight of isopropyl myristate (hereinafter, IPM) as a plasticizer, 4.0 parts by weight of polyoxyethylene (7) oleyl ether (hereinafter BO-7) as a surfactant, 3.0 parts by weight of loxoprofen sodium (a drug forming a water-soluble salt) as a drug, and 3.0 parts by weight of a flavonoid compound (quercetin, apigenin, or myricetin) shown in Table 1 were weighed. These materials were stirred and sufficiently defoameded. Thus, a drug-containing adhesive dispersion was prepared.

This drug-containing adhesive dispersion was spread and dried on a polyester release film to form a drug-containing adhesive layer with a thickness of about 60 µm. The drug-containing adhesive layer was transferred to a laminate-type support made of a 6-µm-thick PET film and a 20 g/m² non-woven fabric, whereby a film was obtained. The obtained film was cut into a rectangle shape with a size of 0.7 cm². Thus, a patch preparation containing loxoprofen sodium was obtained.

### (Comparative Examples 1 and 2)

A patch preparation containing loxoprofen sodium was obtained in the same manner as in Example 1 except that the composition was changed as shown in Table 1. Comparative Example 2 employed curcumin, a polyphenol other than a flavonoid compound.

**[Table 1]**

| | | Example (parts by weight) | | | Comparative Example (parts by weight) | |
|---|---|---|---|---|---|---|
| | | 1 | 2 | 3 | 1 | 2 |
| Mixture of adhesive polymer and tackifier | PIB blend | 63.0 | 63.0 | 63.0 | 65.1 | 63.0 |
| Plasticizer | [PM | 27.0 | 27.0 | 27.0 | 27.9 | 27.0 |
| Surfactant | BO-7 | 4.0 | 4.0 | 4.0 | 4.0 | 4.0 |
| Drug | Loxoprofen sodium | 3.0 | 3.0 | 3.0 | 3.0 | 3.0 |
| Flavonoid compound | Quercetin | 3.0 | - | - | - | - |
| | Apigenin | - | 3.0 | - | - | - |
| | Mvricetin | - | - | 3.0 | - | - |
| Polyphenol other than flavonoid compound | Curcumin | - | - | - | - | 3.0 |

### (Examples 4 to 6, Comparative Examples 3 and 4)

A patch preparation containing HER2/neu-A24 peptide was obtained in the same manner as in Example 1 except that the composition was changed as shown in Table 2. Comparative Example 4 employed curcumin, a polyphenol other than a flavonoid compound.

**[Table 2]**

| | | Example (parts by weight) | | | Comparative Example (parts by weight) | |
|---|---|---|---|---|---|---|
| | | 4 | 5 | 6 | 3 | 4 |
| Mixture of adhesive polymer and tackifier | PIB blend | 58.1 | 58.1 | 58.1 | 60.2 | 58.1 |
| Plasticizer | IPM | 24.9 | 24.9 | 24.9 | 25.8 | 24.9 |
| Surfactant | BO-7 | 4.0 | 4.0 | 4.0 | 4.0 | 4.0 |
| Drug | HER2/neu A24 peptide | 10.0 | 10.0 | 10.0 | 10.0 | 10.0 |
| Flavonoid compound | Quercetin | 3.0 | - | - | - | - |
| | Apigenin | - | 3.0 | - | - | - |
| | Myricetin | - | - | 3.0 | - | - |
| Polyphenol other than flavonoid compound | Curcumin | - | - | - | - | 3.0 |

### (Examples 7 to 9, Comparative Examples 5 to 7)

A patch preparation containing an acidic drug or a basic drug was obtained in the same manner as in Example 1 except that the composition was changed as shown in Table 3.

**[Table 3]**

| | | Example (parts by weight) | | | Comparative Example (parts by weight) | | |
|---|---|---|---|---|---|---|---|
| | | 7 | 8 | 9 | 5 | 6 | 7 |
| Mixture of adhesive polymer and tackifier | PIB blend | 63.0 | 63.0 | 63.0 | 65.1 | 65.1 | 65.1 |
| Plasticizer | IPM | 27.0 | 27.0 | 27.0 | 27.9 | 27.9 | 27.9 |
| Surfactant | BO-7 | 4.0 | 4.0 | 4.0 | 4.0 | 4.0 | 4.0 |
| Acidic drug | Aspirin | 3.0 | - | - | 3.0 | - | - |
| Basic drug | Imiquimod | - | 3.0 | - | - | 3.0 | - |
| | Epinephrine | - | - | 3.0 | - | - | 3.0 |
| Flavonoid compound | Quercetin | 3.0 | 3.0 | 3.0 | - | - | - |

### (Example 10, Comparative Example 8)

A patch preparation free from a surfactant was obtained in the same manner as in Example 1 except that the composition was changed as shown in Table 4.

**[Table 4]**

| | | Example (parts by weight) | Comparative Example (parts by weight) |
|---|---|---|---|
| | | 10 | 8 |
| Mixture of adhesive polymer and tackifier | PIB blend | 65.8 | 67.9 |
| Plasticizer | IPM | 28.2 | 29.1 |
| Drug | Loxoprofen sodium | 3 | 3 |
| Flavonoid compound | Quercetin | 3 | 0 |

### (Examples 11 to 15, Comparative Examples 9 to 13)

A solution in toluene of a mixture of polyisobutylene and a tackifier (a mixture of 24 parts by weight of polyisobutylene B200 (viscosity average molecular weight: 4,000,000), 36 parts by weight of polyisobutylene B12 (viscosity average molecular weight: 55,000), and 40 parts by weight of alicyclic saturated hydrocarbon resin ARKON P-100, each in terms of the solid content; hereinafter referred to as PIB blend) or sodium polyacrylate adhesive was weighed in an amount (parts by weight) shown in Table 5 calculated based on the solid content in the composition. Subsequently, a plasticizer (IPM, IPP, or liquid paraffin) in an amount (parts by weight) shown in Table 5, 3.0 parts by weight of loxoprofen sodium (a drug forming a water-soluble salt) as a drug, a flavonoid compound (quercetin) in an amount (parts by weight) shown in Table 5 were weighed. These materials were stirred and sufficiently defoamed. Thus, a drug-containing adhesive dispersion was prepared.

This drug-containing adhesive dispersion was stretched and dried on a polyester release film to form a drug-containing adhesive layer with a thickness of about 60 µm. The adhesive layer was transferred to a laminate-type support made of a 6-µm-thick PET film and a 20 g/m² non-woven fabric, whereby a film was obtained. The obtained film was cut into a rectangle shape with a size of 0.7 cm². Thus, a patch preparation containing loxoprofen sodium was obtained.

**[Table 5]**

| | | Example (parts by weight) | | | | | Comparative Example (parts by weight) | | | | |
|---|---|---|---|---|---|---|---|---|---|---|---|
| | | 11 | 12 | 13 | 14 | 15 | 9 | 10 | 11 | 12 | 13 |
| Mixture of adhesive polymer and tackifier | PIB blend | 65.8 | 65.8 | | | | 67.9 | 67.9 | | | |
| Adhesive | pAANa adhesive | | | 65.8 | 65.8 | 65.8 | | | 67.9 | 67.9 | 67.9 |
| Plasticizer | IPM | | | 28.2 | | | | | 29.1 | | |
| | IPP | 28.2 | | | 28.2 | | 29.1 | | | 29.1 | |
| | Liquid paraffin | | 28.2 | | | 28.2 | | 29.1 | | | 29.1 |
| Drug | Loxoprofen sodium | 3 | 3 | 3 | 3 | 3 | 3 | 3 | 3 | 3 | 3 |
| Flavonoid compound | Quercetin | 3 | 3 | 3 | 3 | 3 | 0 | 0 | 0 | 0 | 0 |

### <Evaluation>

The patch preparations obtained in the examples and comparative examples were subjected to the following evaluation.

### Skin penetration property test

A mouse skin penetration property test below was performed to evaluate the skin penetration properties of the drug contained in the patch preparations.

A skin sampled from the back of a 9-week-old C57/BL6♀ was immersed for two hours in a phosphate buffer (hereinafter referred to as receptor fluid) containing an enzyme inhibitor dissolved therein. The back skin after immersion was fixed to a Franz permeation cell, and excess droplets were wiped out. Each of the patch preparations obtained in the examples and comparative examples was applied to the back skin, and the Franz permeation cell was filled with the receptor fluid. Twenty-four hours later, the receptor fluid was recovered and filtered through a PTFE membrane filter. Thereafter, the drug content (drug penetration amount) in the receptor fluid was determined by the UPLC/MS method.

Figs. 2, 3, 4, and 5 show the results of the penetration property test performed on the patch preparations obtained in the examples and comparative examples.

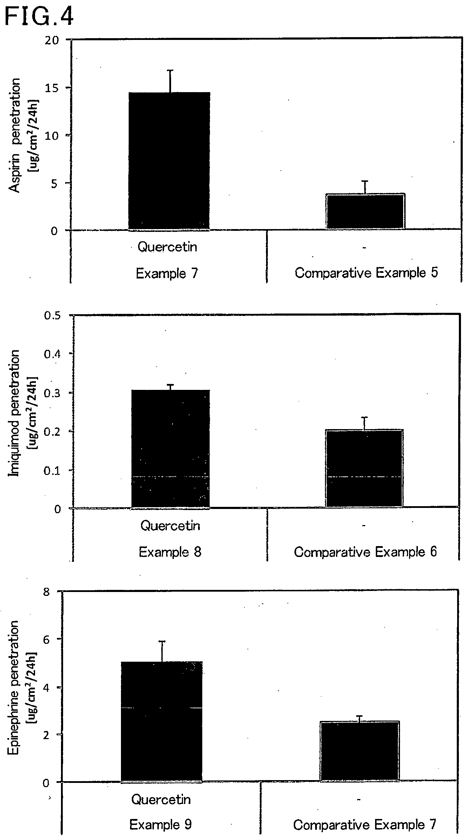

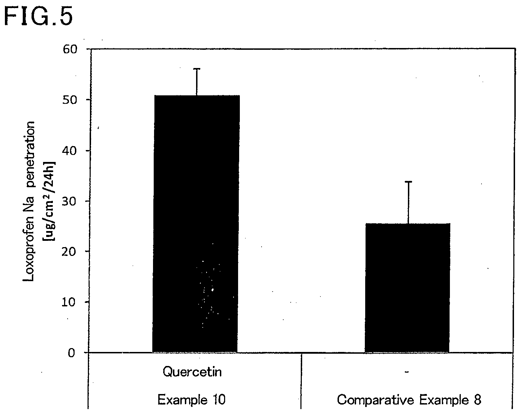

In the patch preparations containing a flavonoid compound obtained in Examples 1 to 3, the skin penetration properties of loxoprofen sodium (a drug forming a water-soluble salt) was improved as compared with those in the patch preparation free from a flavonoid compound obtained in Comparative Example 1 and the patch preparation containing a polyphenol other than a flavonoid compound obtained in Comparative Example 2 (Fig. 2). The same tendency was observed in the cases where HER2/neu-A24 peptide was used as a drug (Fig. 3, Examples 4 to 6, Comparative Examples 3 and 4). Even when the drug used was aspirin, an acidic drug, or imiquimod or epinephrine, a basic drug, the skin penetration properties were improved because of the use of quercetin as a flavonoid compound (Fig. 4, Examples 7 to 9, Comparative Examples 5 to 7). Even in the case of the patch preparation free of BO-7 as a surfactant, the skin penetration properties were improved because of the use of quercetin as a flavonoid compound (Fig. 5, Example 10, Comparative Example 8) . It is to be noted that patch preparations containing a flavonoid compound exhibit improved skin penetration properties than those free from a flavonoid compound even if a different type of plasticizer is used.

### INDUSTRIAL APPLICABILITY

The present invention provides a skin penetration-accelerating composition that eliminates the need for forming a drug into a particle structure and dramatically improves the skin penetration properties of a drug without breaking the skin tissue. The present invention also provides a preparation for transdermal administration containing the skin penetration-accelerating composition and a patch preparation containing the skin penetration-accelerating composition.

### REFERENCE SIGNS LIST

1 patch preparation (matrix-type patch preparation) of the present invention
2 release liner
6 support
7 drug-containing adhesive layer

## Claims

1. A skin penetration-accelerating composition comprising:
a flavonoid compound,
the composition being used for accelerating skin penetration of a drug.

2. The skin penetration-accelerating composition according to claim 1,
wherein the flavonoid compound is a compound having a flavone skeleton represented by Formula (1):

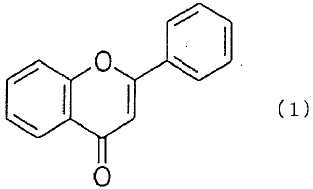

3. The skin penetration-accelerating composition according to claim 2,
wherein the compound having a flavone skeleton represented by Formula (1) is at least one compound selected from the group consisting of quercetin, apigenin, and myricetin.

4. A preparation for transdermal administration comprising:
a drug; and
the skin penetration-accelerating composition according to claim 1, 2, or 3.

5. A patch preparation comprising:
a drug-containing layer that contains a drug and the skin penetration-accelerating composition according to claim 1, 2, or 3.

6. The patch preparation according to claim 5,
wherein the drug-containing layer is a drug-containing adhesive layer, and
the drug-containing adhesive layer is provided on one surface of a support.
